# EUROPÄISCHE PATENTSCHRIFT

(11) **EP 0 915 844 B1**
(45) Veröffentlichungstag und Bekanntmachung des Hinweises auf die Patenterteilung: **26.04.2006**
(21) Anmeldenummer: 97933675.7
(22) Anmeldetag: 14.07.1997
(51) Int. Cl.: C07D 207/22, C12P 17/10

(54) **VERFAHREN ZUR HERSTELLUNG VON 3-PYRROLIN-2-CARBONSÄURE-DERIVATEN**
PROCESS FOR PREPARING 3-PYRROLINE-2-CARBOXYLIC ACID DERIVATIVES
PROCEDE DE PREPARATION DE DERIVES D'ACIDE 3-PYRROLINE-2-CARBOXYLIQUE

(30) Priorität: 26.07.1996 DE 19630082
(43) Veröffentlichungstag der Anmeldung: 19.05.1999
(73) Patentinhaber: Abbott GmbH & Co. KG, 65205 Wiesbaden (DE)
(72) Erfinder: MACK, Helmut, D-67067 Ludwigshafen (DE); PFEIFFER, Thomas, D-67459 Böhl-Iggelheim (DE); SEITZ, Werner, D-68723 Plankstadt (DE); ZIERKE, Thomas, D-67459 Böhl-Iggelheim (DE); BALKENHOHL, Friedhelm, D-67117 Limburgerhof (DE); LANGE, Udo, D-67063 Ludwigshafen (DE)
(74) Vertreter: Grünecker, Kinkeldey, Stockmair & Schwanhäusser Anwaltssozietät
(86) Internationale Anmeldenummer: PCT/EP1997/003752
(87) Internationale Veröffentlichungsnummer: WO 1998/004523

(56) Entgegenhaltungen:
- FR-A- 1 462 258
- FR-A- 1 462 259
- US-A- 4 066 658
- US-A- 4 501 901
- J.K. THOTTATHIL ET AL.: "Lithium diphenylcuprate reactions with 4-tosyloxy-L-prolines; an interesting stereochemical outcome." TETRAHEDRON LETTERS, Bd. 27, Nr. 2, 1986, OXFORD GB, Seiten 151-154, XP002044769
- D.R. KRONENTHAL ET AL.: "Srereospecific Friedel-Crafts alkylation of benzene with 4-mesyloxy-L-prolines.A new synthesis of 4-phenylprolines" TETRAHEDRON LETTERS, Bd. 31, Nr. 9, 1990, OXFORD GB, Seiten 1241-1244, XP002044770

## Beschreibung

Die vorliegende Erfindung betrifft ein neues Verfahren zur Herstellung von Pyrrolin-2-carbonsäure-Derivaten.

Der Austausch von Prolin durch 3,4-Dehydroprolin in biologisch aktiven Peptiden oder Peptidomimetika bewirkt selten einen Verlust an Aktivität (A.M. Felix et al. Int. J. Pept. Prot. Res. 10, 299 (1977); C.R. Botos et al. J. Med. Chem. 22, 926 (1979); G.H. Fisher, W. Ryan, FEBS Lett. 107, 273 (1979)) sondern steigert in einigen Fällen die Wirkung unter gleichzeitiger Verminderung der Toxizität (G.H. Fisher, W. Ryan FEBS Lett. 107, 273 (1979); S. Natarajan et al., in Peptide, Structure and Biological Function, E. Gross, J. Meienhofer, Eds., Pierce Chemical Company, 1979, p. 463).

Die Synthese N-geschützter 3,4-Dehydroproline im technischen Maßstab erfordert nach den literaturbekannten Verfahren einen sehr großen Aufwand, wie beispielsweise die thermische cis-Eliminierung des S-Methyl-xanthogenats aus Hydroxyprolin nach Tchugaeff zeigt. Die Nachteile dieses Verfahrens liegen im Umgang mit großen Mengen Methyljodid, sowie in der Entstehung von Methylmercaptan und Kohlenoxysulfid (J.-R. Dormay et al., Angew. Chem. 92, 761 (1980); Houben-Weil, Methoden der Organischen Chemie, Bnd. 5/1b, 126 (1972)).

Auch die Reduktion der Pyrrol-2-carbonsäure mit Phosphoniumjodid in rauchender Jodwasserstoffsäure ist wegen der Handhabung eines großen Überschusses an gasförmigem Jodwasserstoff sowie einer deutlichen Ausbeuteverminderung und einsetzender Polymerisation bei großen Umsetzungen problematisch (J.W. Scott et al., Synth. Commun. 10(7), 529 (1980)). Unter deutlich milderen Bedingungen dagegen verläuft die Eliminierung des Boc-geschützten 4-Phenylseleninylprolinmethylesters (J.-R. Dormay, Synthesis 9, 753 (1982). Die Eliminierung erfolgt bei Raumtemperatur und führt mit hoher Selektivität zum Δ³-Olefin. Thermische cis-Eliminierungen liefern erhebliche Anteile des isomeren Δ⁴-Olefins. Jedoch ist auch die Eliminierung der Selenoxide wegen anfallender, toxischer selenhaltiger Rückstände, die gerade bei Umsetzungen im Technikumsmaßstab teuer entsorgt werden müssen, sowie die Addition der zuvor abgespaltenen Selenigensäure an die Doppelbindung nachteilig, besonders wenn es sich um Arzneimittelwirkstoffe handelt, in denen schon kleinste Mengen selenhaltiger Verbindungen zu toxischen Eigenschaften führen.

Aus N-substituiertem 3-Methylsulfonyloxy-pyrrolidin konnten geringe Mengen 3-Pyrrolin erhalten werden (T. Uno et al., J. Heterocycl. Chem. 24, 1025 (1987)). Die Eliminierung von Sulfonaten wie z.B. Methylsulfonat zur Herstellung von 3-Pyrrolin-2-carbonsäurederivaten wurde bisher nicht beschrieben.

Die genannten literaturbekannten Verfahren zur Herstellung von 3-Pyrrolin-2-carbonssäure-Derivaten eignen sich nicht für technische Synthesen.

Die vorliegende Erfindung betrifft ein Verfahren zur Herstellung von 3-Pyrrolin-2-carbonsäure-Derivaten der Formel I 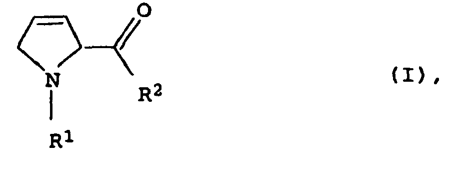 in der
- R¹: H, C₁-C₆-Alkyl, Benzyl, am Phenyl substituiertes Benzyl, Allyloxycarbonyl, C₁-C₆-Alkyloxycarbonyl, Benzyloxycarbonyl, worin der Benzylrest durch OCH₃-Reste substituiert sein kann, oder C₁-C₄-Alkylcarbonyl bedeutet oder
- R¹ und: ein über den C-Terminus verknüpfter Rest einer Aminosäure,die am Stickstoff alkyliert oder acyliert sein kann, bedeutet
- R²: OH, C₁-C₄-Alkyloxy, Benzyloxy oder eine NR³R⁴-Gruppe ist,
worin R³ und R⁴ unabhängig voneinander H, C₁-C₄-Alkyl, Benzyl, Phenyl oder Pyridyl bedeuten, worin die Aromaten in R³ und R⁴ mit bis zu drei gleichen oder verschiedenen Substituenten, ausgewählt aus der Gruppe bestehend aus Methyl, Methoxy, Hydroxy, Cyano oder Halogen substituiert sein können,
welches darin besteht, daß man aus einem Sulfonat der Formel II 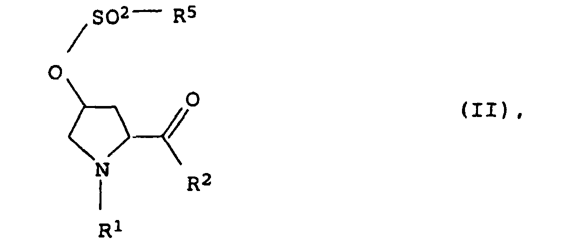 worin R¹ und R² die oben beschriebene Bedeutung besitzen und R⁵ C₁-C₆-Alkyl, Benzyl, Trifluormethyl, Naphthyl oder Phenyl, welches gegebenenfalls mit Resten aus der Gruppe Methyl, Nitro, Halogen substituiert sein kann, bedeutet, den Sulfonsäurerest mit Hilfe einer Base eliminiert.

Als Rest R¹ sind C₁-C₄-Alkylcarbonyl, Benzyl, am Phenyl substituiertes Benzyl, C₁-C₆-Alkyloxycarbonyl und Benzyloxycarbonyl bevorzugt. Falls der Benzyloxycarbonylrest durch OCH₃ substituiert ist, so trägt er vorzugsweise eine Methoxygruppe in der p-Stellung. Besonders bevorzugt ist der C₁-C₆-Alkyloxycarbonylrest.

Bevorzugte Reste R² sind OH und C₁-C₄-Alkoxy.

Bevorzugte Reste R⁵ sind C₁₋₆-Alkyl und Benzyl, insbesondere C₁₋₄-Alkyl.

Die Verbindungen I besitzen ein asymmetrisches C-Atom, die Verbindungen II zwei asymmetrische C-Atome im 5-Ring. Verbindungen der Formel II können als Racemate, Diastereomerengemische, diastereomerenreine und auch als enantiomerenreine Verbindungen eingesetzt werden. Die Verbindungen I können daher je nach stereochemischem Aufbau der als Edukte eingesetzten Verbindungen II und Reaktionsbedingungen als Racemate oder in optisch aktiver Form erhalten werden.

Verbindungen der Formel II lassen sich nach literaturbekannten Methoden herstellen (z.B. D.J. Abraham, M. Mokotoff, L. Sheh, J. E. Simmons, J. Med. Chem. 26(4), 549 (1983)).

Die Eliminierung des Sulfonsäurerests, d.h. der -O-SO₂-R⁵-Gruppe, aus optisch aktiven Verbindungen der Formel II verläuft unter Racemisierung, wenn R² C₁-C₄-Alkoxy oder Benzyloxy ist. Man erhält so racemische Ester des 3,4-Dehydroprolins, die durch nachfolgende enzymatische Racematspaltung sowohl einen Zugang zu D- als auch zu L-3,4-Dehydroprolinderivaten eröffnen (Verfahren A):

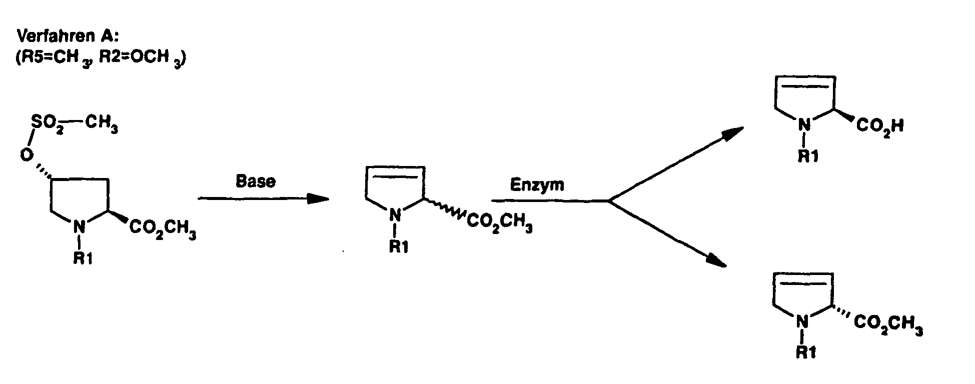

Eine besonders bevorzugte Ausführungsform des Verfahrens besteht darin, daß bei Einsatz von Verbindungen der Formel II, worin R² OH und die absolute Konfiguration des Carbonsäurerests festgelegt ist, diese also entweder der R- oder der S-Konfiguration entspricht, die entsprechenden Carbonsäuren der Formel I racemisierungsfrei erhalten werden können. (Verfahren B):

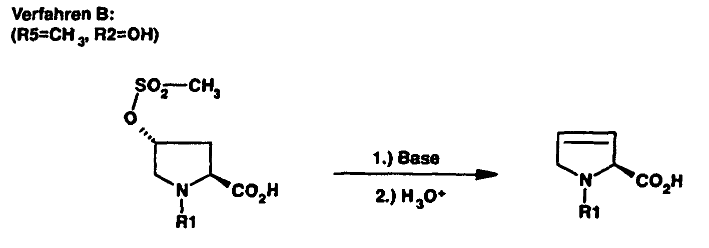

Für die Eliminierungsreaktionen A und B sind nicht-protische Lösungsmittel geeignet, insbesondere DMF, Dioxan, THF, DME, DMSO, CH₃CN, wobei das Lösungsmittel gegebenenfalls kleine Mengen Wasser oder Alkohol enthalten darf.

Es werden, je nach Verfahren, 1,0 bis 1,5 Äquivalente Base (Verfahren A) bzw. 2,0 bis 3,0 Äquivalente Base (Verfahren B) eingesetzt, wobei als Basen Hydride, Amide und Alkoholate des Lithiums, Natriums, Kaliums, Rubidiums, Caesiums, Calciums oder Magnesiums, bevorzugt aber die des Natriums und Kaliums in Betracht kommen. Vorzugsweise werden Natriumalkoholate als Basen eingesetzt, worin als Alkoholatreste primäre, sekundäre und tertiäre Alkohole in Betracht kommen. Es können auch Diole, Triole, Etheralkohole des Typs Tri-, Di- oder Monoethylenglykolmonoether oder Aminoalkohole eingesetzt werden. Bevorzugt seien genannt: Triethylenglykolmonomethylether, Diethylenglykolmonomethylether-oder Ethylenglykolmonomethylether, Dimethylaminoethanol oder 2-[2-(Dimethylamino)-ethoxy]-ethanol.

Die Eliminierung der Sulfonatgruppe erfolgt bereits bei einer Temperatur von -20°C. Die Reaktion kann allgemein bei Temperaturen zwischen -20°C und +100°C durchgeführt werden. Vorteilhaft wird sie bei Temperaturen zwischen-10°C und 60°C durchgeführt. Die Eliminierung der Sulfonatgruppe aus den entsprechenden Estern nach Verfahren A verläuft am α-Kohlenstoffatom des dabei entstehenden 3,4-Dehydroprolinesters schon bei -20°C unter Racemisierung.

Überraschend läßt sich das Verfahren nach der besonders bevorzugten Variante des Verfahrens B mit Edukten der Formel II, wo R² OH und der Carbonsäurerest entweder R- oder S-konfiguriert ist, nahezu racemisierungsfrei durchführen. Als Basen werden in dieser Variante bevorzugt Hydride, primäre Alkoholate, primäre Etheralkoholate oder primäre Aminoalkoholate eingesetzt. Besonders bevorzugt kommen 2-Methoxyethanolat, 2-(2-Methoxyethoxy)-ethanolat oder 2-(2-(Dimethylamino)-ethoxy]-ethanolat zum Einsatz. Bevorzugt werden 2,0 bis 2,5 Äquivalente Base pro Äquivalent Edukt eingesetzt. Der bevorzugte Temperaturbereich für die Reaktion nach dem Verfahren B liegt zwischen -10°C und +25°C.

Die zur Eliminierung verwendete Base kann in fester Form in die Reaktionsmischung eingebracht werden, sie kann aber auch vor der Reaktion in situ hergestellt werden. Ist z.B. die für die Eliminierung verwendete Base Natrium-2-methoxyethanolat, so kann man diese Base vorteilhaft in situ darstellen, wenn man den entsprechenden Alkohol zu einer Lösung bzw. Suspension eines Natriumsalzes einer stärkeren Base wie z.B. Natriumhydrid, Natrium-tert.-butylat oder Natrium-bis-(trimethylsilyl)-amid zutropft.
Die Reaktion kann in semi-batch Fahrweise entweder unter Zulauf der Basenlösung zu dem gelösten Edukt der Formel II oder vorzugsweise unter Zulauf einer Lösung des Edukts II zu der vorgelegten Basenlösung bzw. -suspension durchgeführt werden.

Die Aufarbeitung der Reaktionsmischung kann durch Destillation, Extraktion, Kristallisation, Chromatographie oder eine Kombination derselben erfolgen.

Aus racemischem 3,4-Dehydroprolin kann entweder mit (+)- oder (-)-Weinsäure das gewünschte Enantiomer isoliert werden (s. J.W. Scott et al., Synthetic Communications 10, 529 (1980) bzw. U.S. 4.111.951), oder nach Herstellung der Boc-geschützten Aminosäure kann die Racematspaltung mit optisch aktivem 1-(4-Nitrophenyl)-ethylamin durchgeführt werden (U.S. 4.066.658, J.-U. Kahl, T. Wieland, Liebigs Ann. Chem. 8, 1445 (1981)).

Nach dem bevorzugten Verfahren B racemisierungsfrei hergestellte N-geschützte 3,4-Dehydroproline können vorteilhaft durch Kristallisation als Ammoniumsalze mit achiralen Aminen aufgereinigt werden. Insbesondere kann man L-Boc-3,4-Dehydroprolin als Diethylammoniumsalz in reiner Form erhalten.

Diese Ammoniumsalze eind bevorzugt Verbindungen der Formel IV 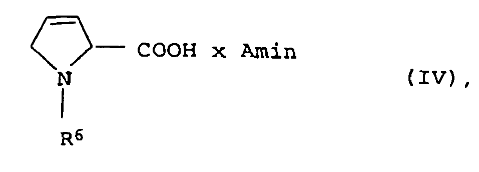 worin R⁶ eine Aminoschutzgruppe und Amin ein Mono-, Di- oder Trialkylamin ist, worin die Alkylreste 1-4-C-Atome enthalten und durch C₅₋₇-Cycloalkylreste ersetzt sein können und deren optisch aktive D- und L-Formen. R⁶ bedeutet vorzugsweise die Boc-Schutzgruppe und "Amin" vorzugsweise Diethylamin oder Dicyclohexylamin.

Nach dem Verfahren A erhaltene Ester lassen sich sehr gut partiell mit Hilfe von Enzymen wie Lipasen, Esterasen und Proteasen, spalten, wobei ein Antipode der freien Säure entsteht, während der andere Antipode in Form des Esters zurückbleibt.

Als Hydrolasen können in dem genannten Verfahren eine Vielzahl von Enzymen eingesetzt werden. Bevorzugt werden Proteasen, Esterasen und insbesondere Lipasen verwendet. Als Lipasen sind vor allem mikrobielle Lipasen gut geeignet, die beispielsweise aus Hefen oder Bakterien isolierbar sind. Weitere besonders gut geeignete Hydrolasen sind die von Novo Nordisk (Enzyme Toolbox) kommerziell erhältlichen Enzyme, insbesondere die Lipasen SP 523, SP 524; SP 525, SP 526 und ®Novozym 435.

Des weiteren können die Lipasen "Chirazyme L1 bis L8", welche kommerziell erhältlich sind (Boehringer Mannheim), vorteilhaft in dem erfindungsgemäßen Verfahren eingesetzt werden.

Auch Esterasen (wie Schweineleberesterase) sind einsetzbar.

Die Enzyme können in nativer oder in immobilisierter Form eingesetzt werden.

Die Esterspaltung wird in einem Puffer bei pH 6-8 und vorzugsweise bei Raumtemperatur durchgeführt.

Das neue Verfahren ermöglicht die Herstellung der Verbindungen I auf eine sehr einfache Weise. Es ist von besonderer Wichtigkeit für die Herstellung von Dehydroprolin-Derivaten, die bislang nur schwierig und z. T. mit schlechter Ausbeute hergestellt werden konnten.

Besonders günstig erhält man nach dem neuen Verfahren optisch aktive N-geschützte 3-Pyrrolin-2-carbonsäurederivate als freie Säure oder in Form eines Esters, aus dem man die freie Säure vorzugsweise enzymatisch freisetzen kann.

Stellt man enzymatisch optisch aktive Säure aus dem Ester her, so bleibt in der Regel ein Antipode des Esters unverändert zurück. Diesen kann man beispielsweise mit Basen racemisieren und erneut der enzymatischen Spaltung unterwerfen.

Der Vorteil der vorliegenden Erfindung besteht darin, daß damit unter milden und gleichzeitig umweltschonenden Reaktionsbedingungen erstmals ein einfacher Zugang zur Herstellung von 3,4-Dehydroprolin-Derivaten in sterisch reiner Form auch im technischen Maßstab ermöglicht wird. Überraschend ist, daß die Eliminierung des Sulfonsäurerestes bereits bei niedrigen Temperaturen durchgeführt werden kann.

Die nach dem neuen Verfahren hergestellten Substanzen sind von großem Interesse. Sie sind z.B. wertvolle Zwischenprodukte zur Herstellung von thrombininhibierenden niedermolekularen Peptidderivaten (vgl. WO 94/29336), bei denen ein Prolinrest gegen einen Dehydroprolinrest ausgetauscht ist. Weiterhin wurde gefunden, daß 3,4-Dehydroprolin ein brauchbares Agens zur Inhibierung der Kollagen-Synthese darstellt (US 4.066.658).

Für die weitere Verarbeitung ist es von besonderem Vorteil, daß man das nach dem erfindungsgemäßen Verfahren erhaltene Rohprodukt ohne weitere Reinigung zur Herstellung der nächsten Zwischenprodukte für die Peptidderivate umsetzen kann, die ihrerseits sehr leicht gereinigt werden können. Diese Zwischenprodukte besitzen die Formel 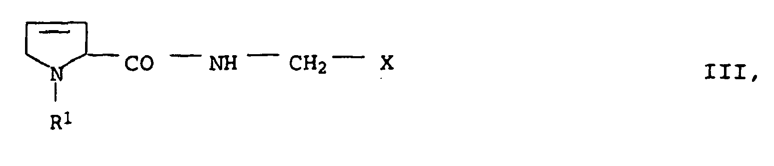 worin R¹ die angegebene Bedeutung besitzt, und X 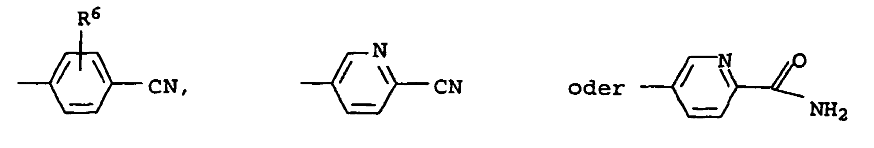 (R⁶ = H, CH₃, OCH₃, OH oder Halogen) bedeutet
Sie lassen sich aus den Verbindungen I herstellen, indem man diese in Gegenwart einer Base wie Triethylamin oder Diisopropylethylamin und einem Kondensationsmittel wie PPA, Pivaloylchlorid oder Dicyclohexylcarbodiimid/Hydroxysuccinimid aktiviert und anschließend mit H₂N-CH₂-X zu Verbindungen der Formel III verknüpft. Die Umsetzung erfolgt zweckmäßig in einem Lösungsmittel wie Dichlormethan, THF, Dioxan, tert.Butyl-methylether, DME oder Acetonitril und wird bei -20 bis +30° durchgeführt.

### Beispiele

In den Beispielen werden folgende Abkürzungen verwendet:
- Bns =: Benzylsulfonyl
- Boc =: tert. Butyloxycarbonyl
- DIPEA =: Diisopropylethylamin
- DME =: Dimethoxyethan
- DMF =: Dimethylformamid
- KOtBu =: Kalium-tert.butylat
- Ms =: Methylsulfonyl
- PPA =: Propylphosphonsäureanhydrid
- Pro =: Prolin
- Pyr =: 3,4-Dehydroprolin
- RT =: Raumtemperatur
- THF =: Tetrahydrofuran

A. Herstellung der Ausgangsstoffe
   a) Boc-(L)-(4-MsO)-Pro-OCH₃ und Boc-(L)-(4-BnsO)-Pro-OCH₃:
      (4R)-N-Boc-4-hydroxy-(L)-prolin-methylester wurde mit Methylsulfonylchlorid zum (4R)-N-Boc-4-methylsulfonyloxy-(L)-prolin-methylester umgesetzt (D.J. Abraham, M. Mokotoff, L. Sheh, J.E. Simmons, J. Med. Chem. 26(4), 549 (1983)). Analog zur Umsetzung mit Ms-chlorid erhielt man mit Benzylsulfonychlorid den (4R)-N-Boc-4-benzylsulfonyloxy-(L)-prolin-methylester nach Kristallisation aus Ethanol mit einer Ausbeute von 76 %. ¹H-NMR (CDCl₃, δ in ppm): 7,40 und 7,28 (s, 5H, Aromat), 4,95 und 4,85 (m, 1H, O-CH), 4,40 (s, 2H, SO₂-CH₂), 4,4 - 4,25 (1H, N-CH), 3,72 und 3,71 (s, 3H, CO₂CH₃), 3,7 - 3,50 (2H, N-CH₂), 2,53 - 1,95 (2H, CH₂, 1,45 und 1,42 (s, 9H, Boc); (2 Rotamere).
   b) (4R)-N-Boc-(4-Ms0)-Pro-OH:
      186 g (575 mMol) des Methylesters Boc-(L)-(4-MsO)-Pro-OCH₃ wurden bei 0°C in 500 ml Dioxan und 1150 ml 1N NaOH 2,5 h hydrolysiert. Nach Extraktion mit Ether wurde die Wasserphase mit 2N Salzsäure auf pH 3 eingestellt und das Produkt mit Essigester extrahiert. Nach Trocknen über Na₂SO₄ und vollständigem Abziehen des Lösungsmittels erhielt man 163 g (92 %) eines gelblichen Öls mit 94 %iger Reinheit. Das Produkt erstarrte langsam zu einem Feststoff;
         [α]_{D}²² = -50,5° (c = 1,01; MeOH); nach Kristallisation aus Diisopropylether
         ¹H-NMR (CDCl₃, δ in ppm): ca. 9 - 8 (COOH), 5,35 - 5,20 (m, 1H, O-CH), 4,60 - 4,40 (1H, N-CH), 3,95 - 3,65 (2H, N-CH₂), 3,08 (s, 3H, SO₂CH₃), 2,85 - 2,25 (2H, CH₂), 1,50 und 1,40 (s, 9H, Boc); (2 Rotamere)
B. Herstellung der Endprodukte

### Beispiel 1

Herstellung von Boc-(D/L)-Pyr-OCH₃:
a) 100 g (309 mMol) Boc-(L)-(4-MsO)-Pro-OCH₃ wurden in 600 ml trockenem DMF gelöst. Bei 0 bis 5°C tropfte man binnen 1 h eine Lösung aus 36,45 g (325 mMol) KOtBu in 300 ml trockenem DMF hinzu und rührte weitere 30 min bei 0 bis 5°C und 2 h bei RT. Anschließend wurde die Mischung auf Eiswasser gegossen, dreimal mit Ether/Essigester 5:1 extrahiert und die organische Phase nochmals mit Wasser gewaschen. Nach dem Trocknen über Na₂SO₄ wurde das Lösungsmittel bei 35°C vollständig abgezogen. Man erhielt 68 g rohen Ester, welcher bei 1,7 mbar und 100 bis 102°C destillierte. Das so erhaltene farblose Öl erstarrte zu einem Feststoff (56 % Ausbeute).
   ¹H-NMR (CDCl₃, δ in ppm): 6,05 - 5,95 (m, 1H, -CH=CH-), 5,80 - 5,67 (m, 1H, -CH=CH-), 5,05 und 4,98 (m, 1H, N-CH), 4,35 - 4,15 (m, 2H, N-CH₂), 3,75 und 3,74 (s, 3H, CO₂CH₃), 1,47 und 1,43 (s, 9H, Boc); (2 Rotamere)
b) Dieselbe Verbindung (Boc-(D/L)-Pyr-OCH₃) erhielt man auch aus dem Benzylsulfonat Boc-(L)-(4-BnsO)-Pro-OCH₃. Aus 5 g (12,5 mMol) des genannten Benzylsulfonats in 50 ml trockenem DMF, welches bei -10°C zu einer Suspension von 0,5 g (12,5 mMol) NaH in 10 ml trockenem DMF getropft und anschließend über Nacht bei RT gerührt wurde, erhielt man nach Aufarbeitung analog zur Eliminierung des Mesylats gemäß a) 2,2 g rohen Ester. Das Produkt ließ sich säulenchromatographisch über Kieselgel reinigen (Laufmittel: Essigester/Hexan 2:3). Wegen des schwachen Chromophors ist eine Destillation aber vorzuziehen.

### Beispiel 2

Herstellung von Boc-(L)-Pyr-OH:
a) Zu 14,5 g 55-65 %igem NaH (ca. 364 m Mol) in 400 ml DME wurden bei RT 50,0 g (161,6 mmol) Boc-(L)-(4-MsO)-Pro-OH, gelöst in 650 ml DME (dem 25 mMol Wasser zugesetzt wurden), in 45 min hinzugetropft, wobei die Temperatur ohne zusätzliche Kühlung auf ca. 30°C anstieg. Die Mischung wurde weitere 15 h bei RT und dann noch 1 h bei 50°C gerührt, anschließend auf Eiswasser gegossen und dreimal mit Ether/Essigester 2:1 gewaschen. Man säuerte die Wasserphase mit 2N Salzsäure auf pH 2 an und extrahierte das Produkt mit Essigester. Nach dem Trocknen über Na₂SO₄ und vollständigem Abziehen des Lösungsmittels erhielt man 36 g Rohsubstanz als gelbliches Ö1, in dem das Produkt zu 70 % enthalten war. Man detektierte ein Produkt-/Edukt-Verhältnis von 97:3 (HPLC:Wasser/Acetonitril 8:2 + 0,1 % TFA; Merck (®) Purospher RP-18e; Detektion bei 210,4 nm) und ein Enantiomerenverhältnis (L):(D) von 90:10. Die Enantiomerenanteile wurden nach Kupplung der Säuregruppe mit einem 3-Picolylaminderivat als entsprechendes Boc-3,4-Dehydroprolyl-(3-picolyl)-amid auf einer chiralen HPLC-Säule detektiert. Vorhergehende Untersuchungen haben gezeigt, daß die Kupplung selbst nahezu racemisierungsfrei verläuft.
   Nach analoger Umsetzung, jedoch mit 3 Äquivalenten NaH und einer Rührzeit von 4 h bei RT, erhielt man 65 % Produkt (Produkt:Edukt = 94:6; (L):(D) = 96:4).
   ¹H-NMR (CDCl₃, δ in ppm) : 10,5-9,5 (COOH), 6,10-5,90 (1H, -CH=CH-), 5,88-5,70 (1H, -CH=CH-), 5,12-4,95 (1H, N-CH), 4,30-4,15 (2H, N-CH₂), 1,55-1,35 (9H, Boc); (2 Rotamere)
   Nach Methoden der klassischen Racematspaltung wurde Boc-3,4-Dehydroprolin und (+)-Dehydroabietylamin aus Aceton zum entsprechenden Ammoniumsalz kristallisiert und ohne weiteres Umkristallisieren, nach Abspaltung des Amins, das Boc-(L)-3,4-Dehydroprolin mit einer Reinheit von 85 % und einem Enantiomerenverhältnis (L) : (D) = 96 : 4 isoliert.
b) 46,2 g Na-tert.-amylat (398,5 mmol) wurden in 150 ml THF vorgelegt. Anschließend wurden bei 10°C 32,9 g 2-Methoxyethanol (429,5 mmol) zugegeben. Danach wurde eine Lösung von 50 g Boc-(L)-(4-MsO)-Pro-OH (159,4 mmol) in 100 ml THF so zugetropft, daß die Innentemperatur 8°-10°C nicht überstieg. Nach Ende der Zugabe wurde 20 h bei 10°C weitergerührt. Nach Zugabe von 300 ml Eiswasser bei 5°-10°C wurde einmal mit 50 ml Methyl-tert.-butylether extrahiert und anschließend mit Salzsäure auf pH 2 angesäuert. Das Rohprodukt wurde mit Methylenchlorid extrahiert und nach Abdampfen des Lösemittels als gelbes Ö1 isoliert.
   Die Auswaage betrug 40,9 g, davon 18 g Boc-(L)-3,4-Dehydroprolin, ermittelt durch mit externem Standard kalbrierter HPLC-Analytik (Anfangsgradient Wasser (0,1% H₃PO₄)/Acetonitril 70:30; Säule: Prodigy (ODS3) 100A; Detektion bei 210 nm). Das Enantiomerenverhältnis (L):(D) von 99:1 wurde ebenfalls durch HPLC-Analytik (Hexan/Isopropanol 8,75:1,25, 0,1% HCOOH; Säule: Chiracel OD; Detektion bei 230 nm) ermittelt.
   In einem unter ähnlichen Bedingungen durchgeführten Ansatz wurden 70 g Boc-(L)-(4-MsO)-Pro-OH (224 mmol) umgesetzt. Nach der Extraktion mit Methylenchlorid wurde das Rohprodukt durch destillativen Lösemitteltausch in 220 ml Methyl-tert.-butylether überführt und anschließend das darin enthaltene Boc-(L)-3,4-Dehydroprolin durch Zugabe von 16,5 g Diethylamin (224 mmol) als Diethylammonium-Salz gefällt.
   Es wurden 23,8 g dieses Salzes erhalten. Nach den oben angegebenen HPLC-Analysemethoden konnte in dem so gefällten Produkt das (D)-Enantiomere nicht nachgewiesen werden.
   ¹H-NMR (DMSO, δ in ppm): 5,86-5,67 (2H, -CH=CH-), 4,6-4,5 (N-CH), 4,1-3,9 (N-CH₂), 2,88-2,7 (4H q, NCH₂CH₃), 1,45-1,25 (9H, Boc 2 Rotamere), 1,2-1,05 (6H, NCH₂CH₃) [α)_{D}²² = -240, 1° (C= 1,08, MeOH) Schmelzpunkt: 130-133°C
c) 10,52 g Bis-(trimethylsilyl)-natriumamid (57,4 mmol) wurden in 25 ml THF vorgelegt, 8,25 g 2-[2-(Dimethylamino)-ethoxy]-ethanol (62 mmol) in 15 ml THF innerhalb von 15 min unter Kühlung zugetropft und 30 min bei RT gerührt. Anschließend tropfte man bei -5°C 7,1 g Boc-(L)-(4-MsO)-Pro-OH (23,0 mmol) in 15 ml THF gelöst innerhalb von 20 min zu, rührte 1 h bei -5°C, 2 h bei 0°C und über Nacht bei RT. Danach wurde der Ansatz auf 125 g Eiswasser gegossen, viermal mit Methyl-tert.-butylether extrahiert, die wäßrige Phase mit 60 ml 10%iger Zitronensäure auf pH 2,2 angesäuert und über Nacht bei RT gerührt. Nach dreimaliger Extraktion der Reaktionslösung mit Methyl-tert.-butylether wurden die gesammelten organischen Phasen nacheinander mit Wasser, gesättigter Kochsalzlösung und Wasser gewaschen, über Magnesiumsulfat getrocknet und im Vakuum eingeengt. Man erhielt 4,1 g Boc-(L)-3,4-Dehydroprolin als Rohprodukt, welches anschließend in 20 ml Methyl-tert.-butylether gelöst und tropfenweise mit einer Lösung von 1,35 g Diethylamin (18,52 mmol) in 10 ml Methyl-tert.-butylether versetzt wurde. Zur Vervollständigung der Salzfällung wurde Petrolether zugesetzt. Nach Absaugen und Trocknen des Produkts erhielt man 4,0 g Boc-(L)-3,4-Dehydroprolin. Aus der Mutterlauge konnte zusätzlich ein Zweitkristallisat mit 0,3 g gewonnen werden, womit die Gesamtausbeute an Wertprodukt 66% betrug.

### Beispiel 3

Herstellung von Boc-(D,L)-Pyr-OH:

Zu 8g 60%igem NaH (200mmol) in 150 ml DME wurden unter Kühlung 13 g Isopropanol (215 mmol) zugetropft. Nach Abklingen der H₂-Entwicklung wurde bei 0°C eine Lösung von 25 g Boc-(L)-(4-MsO)-Pro-OH (80 mmol) in 100 ml DME zugegeben. Nach 1 h bei 0°C wurde 20 h auf 20°C erwärmt und anschließend 150 ml Wasser zugegeben. Nach einmaligem Extrahieren mit Methyl-tert.-butylether wurde mit Salzsäure auf pH 2 angesäuert und mit Methylenchlorid extrahiert. Die Auswaage betrug 17 g. Das Enantiomerenverhältnis (L):(D) wurde via HPLC-Anaylse (Hexan/Isopropanol 8,75:1,25, 0,1% HCOOH; Säule: Chiracel OD; Detektion bei 230 nm) mit 57:43 bestimmt.

### Beispiel 4

Enzymatische Spaltung von Boc-(D/L)-Pyr-OCH₃ zu Boc-(L)-Pyr-OH:

5,68 g (25 mMol) Boc-(D/L)-Pyr-OCH₃ wurden in 100 ml Phosphat-Puffer (pH 7,0) und 15 ml THF mit 3,12 g ®Novozym 435 24 h bei RT geschüttelt. Durch Zugabe von 1N NaOH wurde dabei der pH-Wert auf den Anfangswert nachreguliert. Am Verbrauch der Natronlauge wurde der Verlauf der Reaktion beobachtet. Der Feststoff wurde abfiltriert und das Filtrat mit 1N NaOH auf pH 10 eingestellt. Der nicht umgesetzte Antipode Boc-(D)-Pyr-OCH₃ wurde mit Essigester/Ether 1:1 extrahiert. Die Wasserphase wurde mit 1N Salzsäure auf pH 1 eingestellt und das Produkt Boc-(L)-Pyr-OH dreimal mit Essigester extrahiert. Man erhielt 2,14 g Produkt, welches sich aus Toluol/ Hexan oder Ether/Hexan kristallisieren ließ. Nach der Kristallisation zeigte das Produkt einen Drehwert von [α]_{D}²² = -273,8° (c = 1,03; Methanol). (Lit.: [α]_{D}²⁵ = -272° (c = 1,0; Methanol) J.-U. Kahl, T. Wieland, Liebigs Ann. Chem. 8, 1445 (1981)) .

### Verwendungsbeispiel 1

Herstellung von H-(L)-Pyr-(6-carboxamido)-3-picolylamid Dihydrochlorid:

Zu 1,5 g rohem Boc-Pyr-OH aus Beispiel 2 in 30 ml Dichlormethan tropfte man bei -10°C 5,3 ml (30,3 mMol) DIPEA, gab nach 5 min 1,58 g (7,0 mMol) (6-Carboxamido)-3-picolylamin Dihydrochlorid und nach weiteren 5 min 5,7 ml (7,9 mMol) PPA (50 %-ige Lösung in Essigester) in 5 ml Dichlormethan hinzu. Man ließ das Reaktionsgemisch sich während 1 h von -10°C auf 0°C erwärmen, verdünnte dann mit Dichlormethan und wusch nacheinander mit gesättigter NaHCO₃-Lösung 5 %-iger Zitronensäure und gesättigter Kochsalzlösung. Nach dem Trocknen der organischen Phase über Na₂SO₄ und vollständigem Abziehen des Lösungsmittels erhielt man 1,8 g rohes Boc-(L)-Pyr-(6-carboxamido)-3-picolylamid, welches in 30 ml 0,9 molarer isopropanolischer HCl 50 min bei 50°C gerührt wurde. Der dabei ausgefallene Niederschlag wurde über eine Filternutsche abgetrennt, in wenig Methanol gelöst, mit Isopropanol gefällt und erneut abgetrennt. Nach dem Trocknen bei 45°C im Vakuum erhielt man 2,0 g H-(L)-Pyr-(6-carboxamido)-3-picolylamid Dihydrochlorid als weißes Pulver (95 % Reinheit); (L) : (D) > 99 : 1.
¹H-NMR (DMSO-d6, δ in ppm) : 10,9 und 8,9 9 (je 1H, -NH₂-⊕), 9,77 (t, 1H, CO-NH), 8,60, 8,10 und 8,00 (je 1H, Aromaten-H), 8,25 und 7, 75 (je 1H, CO-NH₂), 6,03 (s, 2H, -CH=CH-) , 5, 10 (1H, N-CH-CO) 4,47 (d, 2H, CH₂) 4,00 (2H, CH₂)

### Verwendungsbeispiel 2

Herstellung von H-(L)-Pyr-(4-CN)-benzylamid Hydrochlorid:

10,0 g rohes Boc-Pyr-OH wurden analog dem Verwendungsbeispiel 1 mit 6,2 g p-Cyano-benzylamin umgesetzt. Man erhielt nach der Aufarbeitung 14,7 g rohes Hoc-(L)-Pyr-(4-CN)-benzylamid, welches in 230 ml 1 molarer isopropanolischer HCl 2 h bei 50°C gerührt wurde. Nachdem die Lösung auf RT abgekühlt war, begann die Substanz langsam auszufallen. Der Feststoff wurde über eine Filternutsche abgetrennt. Man erhielt 3,7 g H-(L)-Pyr-(4-CN)-benzylamid Hydrochlorid als weißes Pulver (96 % Reinheit; (L) : (D) > 99 : 1). 1H-NMR (DMSO-d6, δ in ppm) : 10,9 und 8,9 (je 1H, -NH₂-⊕), 7,82 und 7,47 (je 2H, Aromaten-H), 6,02 (s, 2H, -CH=CH-), 5,10 (1H, N-CH-CO) 4,45 (d, 2H, CH₂) 4,02 (2H, CH₂)

## Patentansprüche

1. Verfahren zur Herstellung von 3-Pyrrolin-2-carbonsäure-Derivaten der Formel I 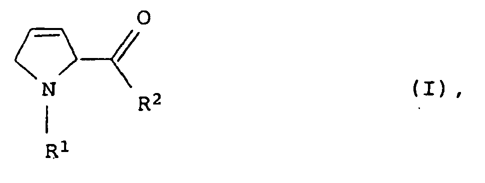 in der
R¹ H, C₁-C₆-Alkyl, Benzyl, am Phenyl substituiertes Benzyl, Allyloxycarbonyl, C₁-C₆-Alkyloxycarbonyl, Benzyloxycarbonyl, worin der Benzylrest durch OCH₃-Reste substituiert sein kann, oder C₁-C₄-Alkylcarbonyl bedeutet oder
R¹ ein über den C-Terminus verknüpfter Rest einer Aminosäure,die am Stickstoff alkyliert oder acyliert sein kann, bedeutet
und
R² OH, C₁-C₄-Alkyloxy, Benzyloxy oder eine NR³R⁴-Gruppe ist,
worin R³ und R⁴ unabhängig voneinander H, C₁-C₄-Alkyl, Benzyl, Phenyl oder Pyridyl bedeutet, worin die Aromaten in R³ und R⁴ mit bis zu drei gleichen oder verschiedenen Substituenten, ausgewählt aus der Gruppe bestehend aus Methyl, Methoxy, Hydroxy, Cyano oder Halogen substituiert sein können,
**dadurch gekennzeichnet, daß** man aus einer Verbindung der Formel II 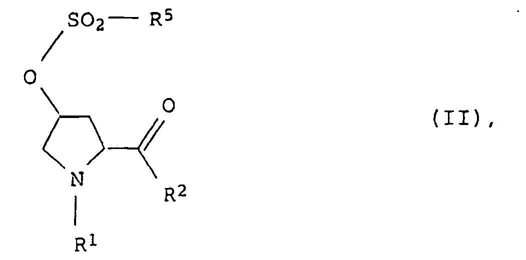 worin R¹ und R² die oben beschriebene Bedeutung besitzen und R⁵ C₁-C₆-Alkyl, Benzyl, Trifluormethyl, Naphthyl oder Phenyl, welches gegebenenfalls mit Resten aus der Gruppe Methyl, Nitro, Halogen substituiert sein kann, bedeutet, mit Hilfe einer Base der Sulfonsäurerest eliminiert.

2. Verfahren gemäß Anspruch 1 zur Herstellung einer optisch aktiven 3-Pyrrolin-2-carbonsäure, **dadurch gekennzeichnet, daß** man aus einer Verbindung der Formel II, worin R¹ und R⁵ die oben beschriebene Bedeutung besitzen und R² C₁-C₄-Alkyloxy oder Benzyloxy ist, den Sulfonsäurerest mit Hilfe einer Base eliminiert und aus dem so erhaltenen racemischen Ester enzymatisch die optisch aktiven freien Säuren freisetzt und diese gegebenenfalls in Form von Ammonium-Salzen isoliert.

3. Verfahren gemäß Anspruch 1 zur Herstellung einer optisch aktiven 3-Pyrrolin-2-carbonsäure, **dadurch gekennzeichnet, daß** man aus einer Verbindung der Formel II, worin der Carbonsäurerest entweder (R)- oder (S)-konfiguriert ist, R¹ und R⁵ die oben angegebene Bedeutung besitzen und R² OH ist, den Sulfonsäurerest mit Hilfe einer Base eliminiert und gewünschtenfalls die so erhaltenen Produkte zur Reinigung und Isolierung in Ammonium-Salze überführt.

4. Verfahren gemäß einem der Ansprüche 1, 2 oder 3 zur Herstellung von 3-Pyrrolin-2-carbonsäure-Derivaten und anschließender Umsetzung zu Verbindungen der Formel III 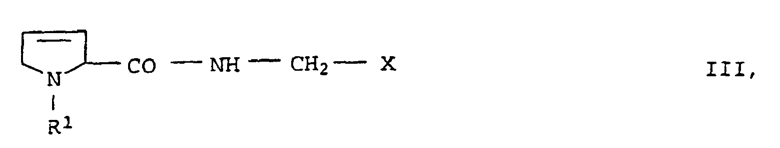 worin
R¹ die angegebene Bedeutung besitzt und X 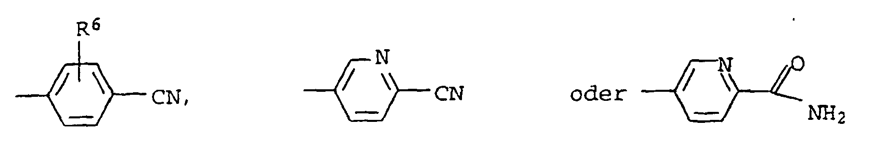 (R⁶ = H, CH₃, OCH₃, OH oder Halogen) bedeutet,
indem die Verbindungen der Formel in Gegenwart einer Base und einem Kondensationsmittel aktiviert und anschließend mit H₂N-CH₂-X zu Verbindungen der Formel III verknüpft werden.

## Claims

1. Process for the preparation of 3-pyrroline-2-carboxylic acid derivatives of the formula I 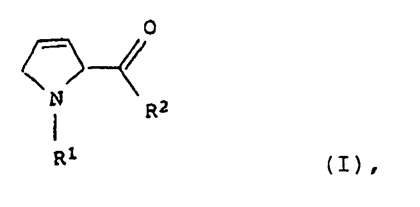 in which
R¹ means H, C₁-C₆-alkyl, benzyl, benzyl substituted on the phenyl, allyloxycarbonyl, C₁-C₆-alkyloxycarbonyl, benzyloxycarbonyl, in which the benzyl residue may be substituted by OCH₃ residues, or C₁-C₄-alkylcarbonyl or
R¹ means a residue of an amino acid which is linked via the C terminus and may be alkylated or acylated on the nitrogen,
and
R² is OH, C₁-C₄-alkyloxy, benzyloxy or an NR³R⁴ group,
in which R³ and R⁴ mutually independently mean H, C₁-C₄-alkyl, benzyl, phenyl or pyridyl, in which the aromatic systems in R³ and R⁴ may be substituted by up to three identical or different substituents selected from the group consisting of methyl, methoxy, hydroxyl, cyano or halogen,
**characterised in that** the sulfonic acid residue is eliminated with the aid of a base from a compound of the formula II 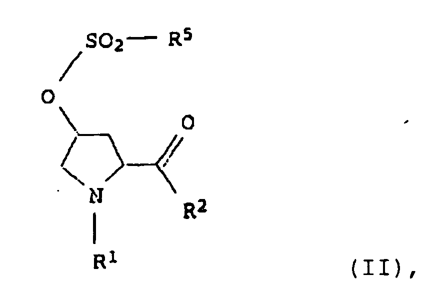 in which R¹ and R² have the meanings described above, and R⁵ means C₁-C₆-alkyl, benzyl, trifluoromethyl, naphthyl or phenyl which may optionally be substituted by residues from the group of methyl, nitro or halogen.

2. Process according to claim 1 for the preparation of an optically active 3-pyrroline-2-carboxylic acid, **characterised in that** the sulfonic acid residue is eliminated with the aid of a base from a compound of the formula II in which R¹ and R⁵ have the meanings described above, and R² is C₁-C₄-alkyloxy or benzyloxy, and the optically active free acids are liberated enzymatically from the resultant racemic ester and said optically active free acids are optionally isolated in the form of ammonium salts.

3. Process according to claim 1 for the preparation of an optically active 3-pyrroline-2-carboxylic acid, **characterised in that** the sulfonic acid residue is eliminated with the aid of a base from a compound of the formula II in which the carboxylic acid residue has either the (R) or the (S) configuration, R¹ and R⁵ have the above-stated meaning, and R² is OH, and, if desired, the resultant products are converted into ammonium salts for purification and isolation.

4. Process according to any one of claims 1, 2 or 3 for the preparation of 3-pyrroline-2-carboxylic acid derivatives and subsequent conversion to yield compounds of the formula III 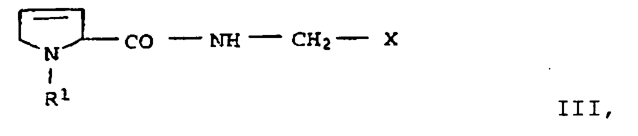 in which
R¹ has the stated meaning and X means 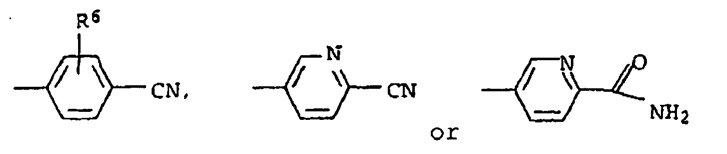 (R⁶ = H, CH₃, OCH₃, OH or halogen),
by activating the compounds of the formula I in the presence of a base and a condensing agent and then linking with H₂N-CH₂-X to yield compounds of the formula III.

## Revendications

1. Procédé de préparation de dérivés d'acide 3-pyrroline-2-carboxylique de formule Ï 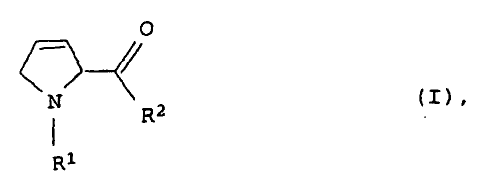 dans laquelle
R¹ représente H, un groupe alkyle en C₁-C₆, benzyle, benzyle substitué sur le radical phényle, allyloxycarbonyle, alkyloxycarbonyle en C₁-C₆, benzyloxycarbonyle, dans lequel le radical benzyle peut être substitué par des résidus OCH₃, ou alkylcarbonyle en C₁-C₄ ou
R¹ représente un résidu d'un acide aminé qui peut être alkylé ou acylé sur l'atome d'azote, relié par la terminaison C
et
R² représente OH, un groupe alkyloxy en C₁-C₄, benzyloxy ou un groupe NR³R⁴,
dans lequel R³ et R⁴ représentent, indépendamment l'un de l'autre, H, un groupe alkyle en C₁-C₄, benzyle, phényle, ou pyridyle, où les aromates dans R³ et R⁴ peuvent être substitués par jusqu'à trois substituants identiques ou différents, choisis dans le groupe constitué du méthyle, méthoxy, hydroxy, cyano ou halogène,
**caractérisé en ce que** l'on élimine d'un composé de formule II 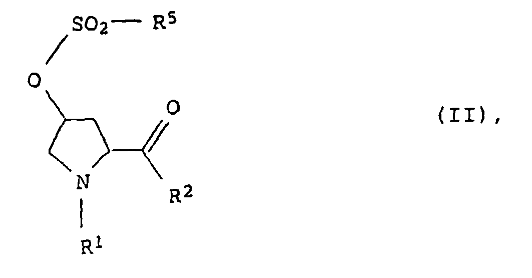 dans laquelle R¹ et R² possèdent la signification susmentionnée et R⁵ représente un groupe alkyle en C₁-C₆, benzyle, trifluorométhyle, naphtyle ou phényle, qui peut être le cas échéant substitué par des radicaux provenant du groupe méthyle, nitro, halogène,
le radical acide sulfonique à l'aide d'une base.

2. Procédé selon la revendication 1 pour la préparation d'un acide 3-pyrroline-2-carboxylique optiquement actif, **caractérisé en ce que** l'on élimine d'un composé de formule II, dans laquelle R¹ et R⁵ possèdent la signification susmentionnée et R² est un groupe alkyloxy en C₁-C₄ ou benzyloxy, le radical acide sulfonique à l'aide d'une base et on libère enzymatiquement les acides libres optiquement actifs à partir de l'ester racémique ainsi obtenu et on isole ceux-ci le cas échéant sous forme de sels d'ammonium.

3. Procédé selon la revendication 1 pour la préparation d'un acide 3-pyrroline-2-carboxylique optiquement actif, **caractérisé en ce que** l'on élimine d'un composé de formule II, dans laquelle le radical acide carboxylique est configuré en (R) ou en (S), R¹ et R⁵ possèdent la signification susmentionnée et R² est OH, le radical acide sulfonique à l'aide d'une base et on transforme en sels d'ammonium les produits ainsi obtenus pour la purification et l'isolation, si on le désire.

4. Procédé selon l'une des revendications 1, 2 ou 3 pour la préparation de dérivés d'acide 3-pyrroline-2-carboxylique et la conversion consécutive en composés de formule III, 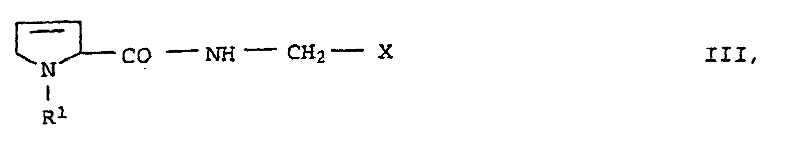 dans laquelle
R¹ possède la signification indiquée et X représente 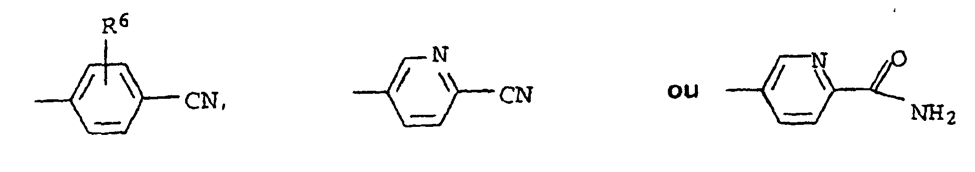 (R⁶ = H, CH₃, OCH₃, OH ou halogène),
en ce que les composés de formule I sont activés en présence d'une base et d'un agent de condensation et sont ensuite reliés avec H₂N-CH₂-X pour donner des composés de formule III.
